# EUROPÄISCHE PATENTSCHRIFT

(11) **EP 2 113 593 B1**
(45) Veröffentlichungstag und Bekanntmachung des Hinweises auf die Patenterteilung: **16.03.2016**
(21) Anmeldenummer: 09157147.1
(22) Anmeldetag: 02.04.2009
(51) Int. Cl.: D04B 1/18, A61F 13/08

(54) **Rundgestrick für die Kompressionstherapie**
Circular knitted fabric for compression therapy
Tricot circulaire pour la thérapie par compression

(30) Priorität: 02.05.2008 DE 102008021998
(43) Veröffentlichungstag der Anmeldung: 04.11.2009
(73) Patentinhaber: Julius Zorn GmbH, 86551 Aichach (DE)
(72) Erfinder: Achtelstetter, Karl, 86453, Dasing-Wessiszell (DE)
(74) Vertreter: Charrier, Rapp & Liebau

(56) Entgegenhaltungen:
- EP-A- 0 261 800
- WO-A-2008/069522
- FR-A- 2 588 890
- US-A- 3 013 420

## Beschreibung

Die Erfindung betrifft ein Rundgestrick für die Kompressionstherapie sowie aus dem Rundgestrick hergestellte Kompressionsartikel für die Kompressionstherapie von Lip- oder Lymphödemen, ulcus cruris venosum und zur Narbentherapie.

Unter einem Lymphödem wird das chronische Anschwellen von Körperextremitäten, verstanden. Die Anschwellung wird durch einen ungenügenden Transport der Lymphflüssigkeit und die daraus resultierende Ansammlung freier eiweißhaltiger Flüssigkeit im Zwischenzellgewebe hervorgerufen. Eine Sonderform ist das Lip-Ödem, welches aufgrund einer Fettverteilungsstörung hervorgerufen wird, die zu einer chronischen Fettansammlung insbesondere im Oberschenkel- und Hüftbereich führt.

Zur Behandlungen von Lymphödemen und Lip-Ödemen werden Entstauungstherapien durchgeführt. Hierbei wird eine manuelle oder operativ unterstützte Lymphdrainage und/oder eine Kompressionstherapie mit Kompressionsverbänden oder Kompressionsstrümpfen angewandt. Häufig werden die Lymphdrainage und die Kompressionstherapie kombiniert. Bei der manuellen Lymphdrainage wird mittels einer speziellen Massagetechnik der Lymphtransport angeregt und Verhärtungen des Gewebes werden gelockert. Um zu verhindern, dass das Ödem nach der Lymphdrainage wieder anschwillt, wird anschließend eine Kompressionsbehandlung mittels Kompressionsverbänden oder Kompressionsstrümpfen durchgeführt.

Für die Kompressionsbehandlung werden in der Regel flachgestrickte Kompressionsartikel verwendet. Es sind hierfür beispielsweise flachgestrickte Kompressionsstrümpfe bekannt, welche Masche für Masche der Körperform folgend gestrickt sind. Das Flachgestrick wird zur Bildung eines Kompressionsstrumpfs mit einer flachen, elastischen Naht zusammen genäht. Im Vergleich zu Rundgestricken, mit denen nahtlose Strümpfe gebildet werden können, weisen Flachgestricke in der Regel eine gleichmäßigere, flächige Kompressionswirkung auf, welche für die Kompressionsbehandlung von Ödemen erforderlich ist. Ein kräftiger, aus einem Flachgestrick gebildeter Kompressionsstrumpf gibt dem Ödem nicht nach und durch Bewegung der zu behandelnden Körperextremitäten entsteht ein hoher Arbeitsdruck, der eine optimale Komprimierung des Gewebes ermöglicht.

Aus der DE. 296 18 426 U1 ist eine strukturierte Auflagefläche für Druckverbände, insbesondere zur Behandlung von Ödemen bekannt, die sich aus einem Grundkörper mit darauf angeordneten, im Wesentlichen gleichförmigen Erhebungen zusammensetzt. Durch die Bewegung des Patienten sollen die Erhebungen relativ zur Körperoberfläche des zu behandelnden Körperteils einen Massageeffekt (Mikromassage) bewirken, wodurch eine Lockerung der Fibrosierungen (Binde- und Fettgewebsverhärtungen und Eiweißeinlagerungen) im Unterhautgewebe sowie eine Aktivierung der Mikrozirkulation und gleichzeitig ein verbesserter Lymphabfluss bewirkt werden soll.

Aus der US 3013420-A ist ein elastisches Gestrick zur Verwendung im Strumpfwarenbereich bekannt, das longitudinal abwechselnd angeordnete Protuberanzen unterschiedlicher Größe und Form aufweist, die ein Oberflächenmuster bilden.

Aus der älteren internationalen Patentanmeldung PCT/KR2007/006207 (WO 2008/069522 A1) ist ein Rundgestrick zur Verwendung in Unterbekleidungsstücken bekannt, welches in engem Kontakt an den Gliedmaßen des Trägers anliegt und dabei deren Form korrigieren bzw. verschönern soll. Das Gestrick weist bogenförmige Erhebungen auf, die ein Oberflächenmuster bilden und bei Kontakt mit der Haut des Trägers einen Massageeffekt ausüben.

Die zur Kompressionstherapie von Ödemen regelmäßig eingesetzten Flachgestrick-Kompressionsartikel in Form von Kompressionsstrümpfen und -ärmeln sind bei den Patienten nicht sehr beliebt, da sie - verglichen mit Rundgestrick-Kompressionsartikeln - weniger bequem und optisch weniger ansprechend sind. Insbesondere die bei Flachgestricken zwangsläufig vorhandene Verbindungsnaht wirkt beim Tragen störend.

Aufgabe der Erfindung ist es daher, ein Gestrick für die Kompressionstherapie von LymphÖdemen und Lip-Ödemen in Form eines angenehmer zu tragenden Rundgestricks bereit zu stellen, welches beim Tragen auf das zu therapierende Körperteil einen ausreichend hohen Kompressionsdruck ausübt und gleichzeitig eine Verbesserung des Lymphabflusses bewirkt. Gelöst wird die Aufgabe mit einem Rundgestrick mit den Merkmalen des Anspruchs 1. Bevorzugte Ausgestaltungen und Verwendungen dieses Rundgestricks sind den Unteransprüchen zu entnehmen.

Das erfindungsgemäße Rundgestrick weist eine musterförmig strukturierte Oberfläche mit Erhebungen auf, die bei Anlage an dem kompressiv zu therapierenden Körperteil einen Flächendruck auf das Körperteil und bei Bewegung einen Massageeffekt ausüben, wobei die strukturierte Oberfläche ein regelmäßiges Muster in Form von Kammern aufweist, die durch die Erhebungen begrenzt werden und in Längsrichtung des Rundgestricks benachbarte Kammern durch Verbindungskanäle miteinander in Verbindung stehen. Die Erhebungen üben bei an einem Körperteil angelegtem Rundgestrick einen Flächendruck auf die Oberfläche des Körperteils aus. Bei Bewegung des Körperteils erfolgt zusätzlich ein Massageeffekt, da die Erhebungen gegenüber der Körperoberfläche bewegt werden und auf dieser entlang gleiten und diese so massieren. Dieser Massageeffekt hat eine lymphabflussfördernde Wirkung. Die durch den Massageeffekt in Bewegung gesetzte Lymphflüssigkeit kann durch die Verbindungskanäle, welche die Kammern miteinander verbinden, in Längsrichtung des Rundgestricks (bildlich gesprochen) durch die Kammern fließen und auf diese Weise zur Niere gefördert werden. Es erfolgt also durch den Massageeffekt und durch den Abfluss der Lymphflüssigkeit durch die Kammern in Längsrichtung L des Rundgestricks eine Lymphdrainage.

Das Rundgestrick ist aus einem Grundgestrick mit einem Strickfaden gebildet, in dem in den Kammern ein dehnbarer Schussfaden 1:1 versetzt hinterlegt ist, wobei der Strickfaden in den Erhebungen Fanghenkel bildet und der Schussfaden an den Stellen, an denen der Strickfaden Fanghenkel bildet, nicht in das Gestrick eingebunden ist. In Erhebungen mit Verlauf in Maschenstäbchenrichtung bildet der Strickfaden nur in jeder zweiten Reihe Fanghenkel und eine Flottung des Schussfaden sowie die darauf folgende Einbindestelle ist um eine Masche seitlich versetzt, so dass eine Flottung des Schussfadens über zwei oder drei Maschen entsteht.

Die Erfindung wird nachfolgend anhand eines Ausführungsbeispiels unter Bezugnahme auf die begleitenden Zeichnungen näher erläutert. Die Zeichnungen zeigen:
- **Fig. 1:**: Maschenbildzeichnung einer ersten Ausführungsform des erfindungsgemäßen Rundgestricks (Fig. 1a) und computergenerierte Musterbilder dieser ersten Ausführungsform des Rundgestricks für den Strickfaden (Fig. 1b) und den Schussfaden (Fig. 1c);
- **Fig. 2:**: Maschenbildzeichnung einer zweiten Ausführungsform des erfindungsgemäßen Rundgestricks (Fig. 2a) und computergeneriertes Musterbild dieser zweiten Ausführungsform für den Schussfaden (Fig. 2b).

Das erfindungsgemäße Rundgestrick besteht aus einem Rechts-Links-Grundgestrick, welches aus einem Strickfaden zusammengesetzt wird, der zur Bildung eines regelmäßigen Musters Fanghenkel bildet. Das Gestrick wird dabei von den Fanghenkeln in Längsrichtung L des Rundgestricks zusammengezogen. In dem Grundgestrick ist ein elastischer Schussfaden 1:1 versetzt hinterlegt. Der Schussfaden wird bevorzugt von Elastofasern gebildet, insbesondere Elastodien, Elasthan oder auch mit Polyamid umwundene Elasthanfäden. Bei dem Strickfaden handelt es sich bevorzugt um einen mit Polyamid, Polyester, Baumwolle, Wolle, Seide oder jedem anderen textilen Material (welches mit elastischen Fäden verarbeitet werden kann) umwundenen Elasthanfaden.

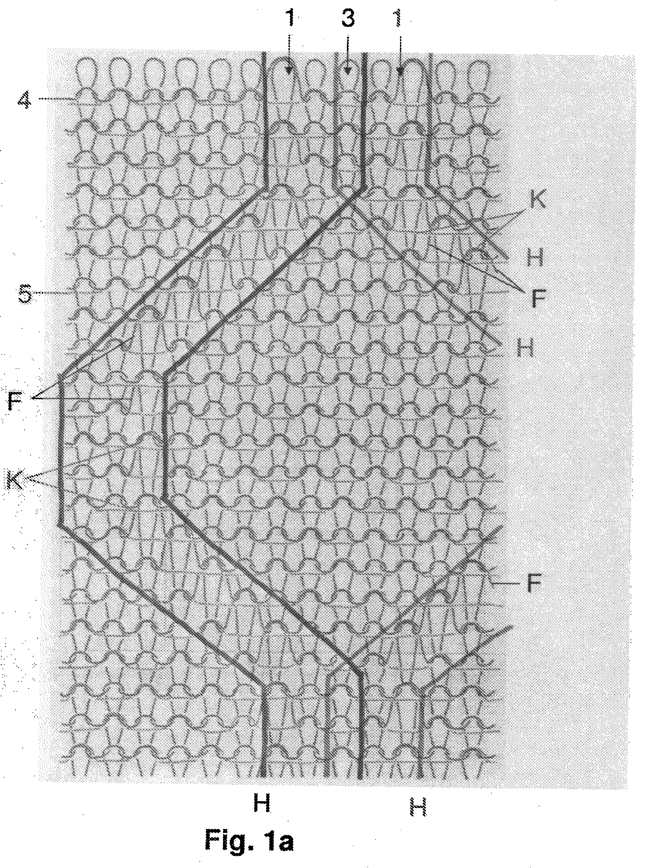

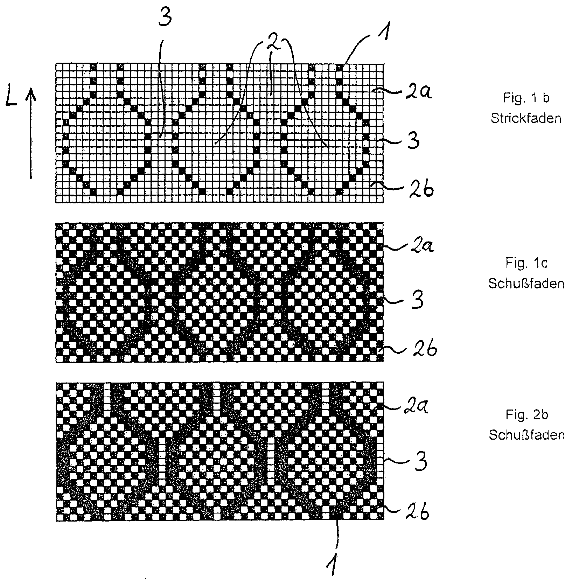

In der **Figur 1** ist eine erste Ausführungsform des erfindungsgemäßen Rundgestricks in Form eines Maschenbildes (Fig. 1a) und in Form eines computergenerierten Musterbildes zeichnerisch dargestellt (Fig. 1b und Fig. 1c). Bei diesem ersten Ausführungsbeispiel des erfindungsgemäßen Rundgestricks ist der Schussfaden 5 an den Stellen, an denen der Strickfaden 4 Fanghenkel F bildet, nicht in das Gestrick eingebunden, sondern flott gelegt. Durch diese Flottung K des Schussfadens 5 zieht der Schussfaden über den Bereich der flott liegenden Fadenstrecke das Gestrick in der Breite, also in Umfangsrichtung des Rundgestricks, zusammen und bewirkt die Kompression.

Das auf die Weise gestrickte Rundgestrick bildet eine musterförmig strukturierte Oberfläche mit Erhebungen 1, wobei diese Erhebungen in den Gestrickbereichen gebildet sind, in denen der Schussfaden flott liegt. In den Bereichen mit der Flottung K des Schussfadens 5 sind die Erhebungen 1 gebildet. In den computergenerierten Musterbildern der Figuren 1b und 1c ist das Flächenmuster mit den Erhebungen 1 zu erkennen. Die Erhebungen 1 umschließen Kammern 2, innerhalb derer das Gestrick keine Erhebungen aufweist. In Längsrichtung L des Rundgestricks benachbarte Kammern 2a, 2b sind durch Verbindungskanäle 3 miteinander verbunden, wobei diese Verbindungskanäle 3 ebenfalls Bereiche ohne Erhebungen im Gestrick darstellen. In Figur 1a sind die Erhebungen durch die Hilfslinien H dargestellt, welche nur zur Verdeutlichung dienen und nicht Bestandteil der Gestrickstruktur sind.

Die Kammern 2 sind rauten- oder - wie in den Musterbildern der Figuren 1b und 1c dargestellt - wabenförmig ausgebildet. Aufgrund der in diesem Muster schräg verlaufenden Fanghenkel F können Fanghenkel in jeder Maschenreihe gebildet werden, da sie in der nächsten Maschenreihe wieder mit einer Masche verstrickt sind. Im vertikalen Bereich des Musters kann nur in jeder zweiten Maschenreihe ein Fanghenkel gebildet werden, da jeder Fanghenkel wieder zu einer Masche abgestrickt werden muss. Dadurch fällt in jeder zweiten Maschenreihe der flott liegende Schussfaden weg.

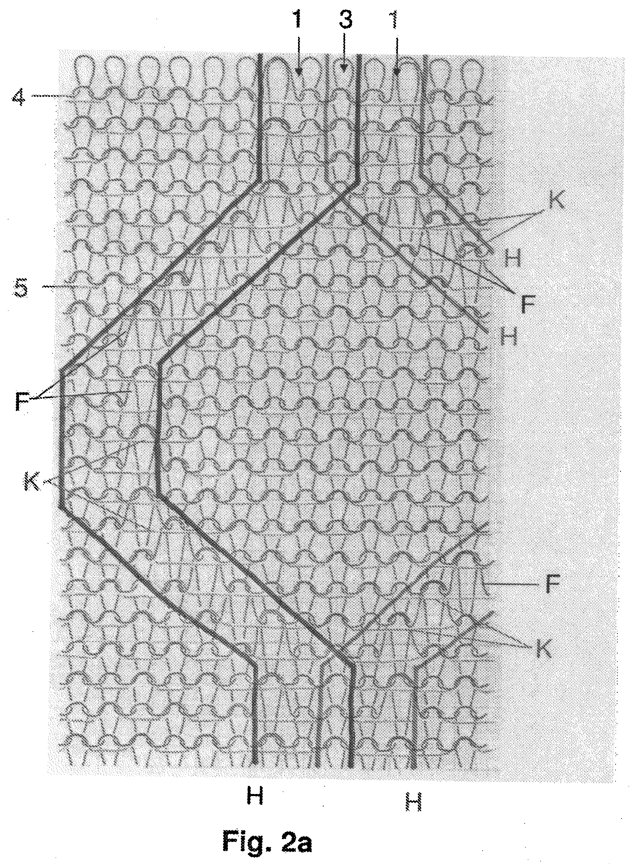

Bei der in Figur 2 gezeigten zweiten Ausführungsform des erfindungsgemäßen Rundgestricks werden die Verbindungskanäle noch deutlicher ausgebildet. Gegenüber der Ausführungsform der Figur 1 erfolgt hier eine Abweichung von der Grundbindung im vertikalen Bereich. Die Flottung K des Schussfadens 5 ist hier um eine Nadel seitlich versetzt. Auch die darauf folgende Einbindestelle wird um eine Nadel seitlich versetzt, so dass eine Flottung K über zwei Nadeln entsteht. An der schmalen Stelle zwischen den so gebildeten Erhebungen 1, welche die Verbindungskanäle 3 zwischen den von den Erhebungen 1 begrenzten Kammern 2 bilden, wird der Schussfaden 5 in jeder Reihe übereinander eingebunden. Die computergenerierten Musterbilder für den Strickfaden (Fig. 1b) sind für beide Ausführungsformen gleich. Die Musterbilder für den Schussfaden unterscheiden sich. In dem Ausführungsbeispiel der Figur 2 kommt das rautenförmige Muster, welches durch die Erhebungen 1 gebildet wird, deutlicher heraus als beim Ausführungsbeispiel der Figur 1 (vgl. Figuren 2b und 1c).

Die Erhebungen 1 üben bei an einem Körperteil angelegtem Rundgestrick einen Flächendruck auf die Oberfläche des Körperteils aus. Bei Bewegung des Körperteils erfolgt zusätzlich ein Massageeffekt, da die Erhebungen 1 gegenüber der Körperoberfläche bewegt werden und auf dieser entlang gleiten und diese so massieren. Dieser Massageeffekt hat eine lymphabflussfördernde Wirkung. Die durch den Massageeffekt in Bewegung gesetzte Lymphflüssigkeit kann durch die Verbindungskanäle 3, welche die Kammern 2 miteinander verbinden, in Längsrichtung des Rundgestricks (bildlich gesprochen) durch die Kammern 2 fließen und auf diese Weise zur Niere gefördert werden. Es erfolgt also durch den Massageeffekt und durch den Abfluss der Lymphflüssigkeit durch die Kammern in Längsrichtung L des Rundgestricks eine Lymphdrainage. Ein aus einem erfindungsgemäßen Rundgestrick hergestellter Kompressionsartikel wird zweckmäßig so getragen, dass die rechte Warenseite auf der Haut aufliegt. Herkömmliche Kompressionsartikel werden normalerweise anders herum getragen. Auf der rechten Warenseite tritt das Muster des erfindungsgemäßen Rundgestricks am stärksten hervor und kann daher auf der Haut einen stärkeren Massageeffekt ausüben.

Das erfindungsgemäße Rundgestrick kann zur Herstellung von Kompressionsartikeln, wie z.B. Kompressionsstrümpfen oder Kompressionsärmeln verwendet werden, welche zur Kompressionstherapie von Lymphödemen eingesetzt werden können. Zur Kompressionstherapie von Lymphödemen können entsprechend aus einem erfindungsgemäßen Rundgestrick hergestellte Kompressionsartikel verwendet werden, wobei der ganze Körper, d.h. insbesondere sämtliche Gliedmaßen, mit solchen Kompressionsartikeln therapiert werden kann.

## Patentansprüche

1. Rundgestrick für die Kompressionstherapie mit einer musterförmig strukturierten Oberfläche mit Erhebungen (1), die bei Anlage an dem kompressiv zu therapierenden Körperteil einen Flächendruck auf das Körperteil und bei Bewegung einen Massageeffekt ausüben, wobei die strukturierte Oberfläche ein regelmäßiges Muster in Form von Kammern (2) aufweist, die durch die Erhebungen (1) begrenzt werden und in Maschenstäbchenrichtung (L) des Rundgestricks benachbarte Kammern (2a, 2b, 2c) durch Verbindungskanäle (3) miteinander in Verbindung stehen, **dadurch gekennzeichnet, dass** das Rundgestrick aus einem Grundgestrick mit einem Strickfaden (4) gebildet ist, in dem in den Kammern (2) ein dehnbarer Schussfaden (5) 1:1 versetzt hinterlegt ist, wobei der Strickfaden (4) in den Erhebungen (1) Fanghenkel (F) bildet und der Schussfaden (5) in den Erhebungen (1) nicht in das Grundgestrick eingebunden ist und der Strickfaden (4) in Erhebungen (1) mit Verlauf in der Maschenstäbchenrichtung nur in jeder zweiten Reihe Fanghenkel (F) bildet und eine Flottung (K) des Schussfaden (5) sowie die darauf folgende Einbindestelle um eine Masche seitlich versetzt ist, so dass eine Flottung (K) des Schussfadens (5) über zwei oder drei Maschen entsteht.

2. Rundgestrick nach Anspruch 1, **dadurch gekennzeichnet, dass** die Kammern (2) rauten- oder wabenförmig ausgebildet sind.

3. Verwendung eines Rundgestricks nach einem der voranstehenden Ansprüche zur Herstellung eines Kompressionsartikels zur Kompressionstherapie von Lip- oder Lymphödemen, ulcus cruris venosum oder zur Narbentherapie.

4. Kompressionsartikel, insbesondere Kompressionsstrumpf oder Kompressionsärmel zur Kompressionstherapie, hergestellt aus einem Rundgestrick nach einem der Ansprüche 1 oder 2.

## Claims

1. Circular knitted fabric for compression therapy having a surface with a patterned texture with raised portions (1) which, when applied to the body part to undergo therapy by compression, exert a surface pressure on the body part and exert a massage effect during movement, wherein the textured surface has a regular pattern in the form of compartments (2) which are bounded by the raised portions (1), and compartments (2a, 2b, 2c) which are adjacent in the direction (L) of the wales of the circular knitted fabric are connected to each other by connecting channels (3), **characterised in that** thecircular knitted fabric is composed of a basic knitted fabric with a knitting yarn (4) in which an elastic weft yarn (5) is deposited in the compartments (2) with an offset of 1:1, wherein the knitting yarn (4) forms tuck loops (F) in the raised portions (1), and the weft yarn (5) in the raised portions (1) is not tied into the basic knitting fabric, and the knitting yarn (4) in raised portions (1) running in the direction of the wales forms tuck loops (F) only in every other row, and a floating stitch (K) of the weft yarn (5) as well as the subsequent tying-in point is laterally offset by one stitch, so that a floating stitch (K) of the weft yarn (5) is formed over two or three stitches.

2. Circular knitted fabric according to claim 1, **characterised in that** the compartments (2) are lozenge-shaped or honeycomb-like.

3. Use of acircular knitted fabric according to one of the preceding claims for the manufacture of a compression article for the compression therapy of lipoedema or lymphoedema, ulcuscrurisvenosum or for scar treatment.

4. Compression article, in particular compression stocking or compression sleeve forcompression therapy, made from a circular knitted fabric according to one of claims 1 or 2.

## Revendications

1. Tricot circulaire pour la thérapie par compression comprenant une surface structurée en forme de trame, pourvue de parties surélevées (1), qui exercent, en cas d'application contre la partie du corps à traiter par compression, une pression de surface sur la partie du corps et un effet massant en cas de déplacement, sachant que la surface structurée présente une trame régulière sous la forme de compartiments (2), qui sont délimités par les parties surélevées (1), et sachant que des compartiments (2a, 2b, 2c) adjacents sont reliés les uns aux autres par des canaux de liaison (3) dans le sens des rangées de mailles (L) du tricot circulaire, **caractérisé en ce que** le tricot circulaire est formé à partir d'un tricot de base présentant un fil à tricoter (4), dans lequel un fil de trame (5) extensible est déposé selon un décalage de 1:1 dans les compartiments (2), sachant que le fil à tricoter (4) forme, dans les parties surélevées (1), des mailles flottées (F) et que le fil de trame (5) n'est pas attaché dans les parties surélevées (1) au tricot de base et que le fil à tricoter (4) dans les parties surélevées (1) s'étendant dans le sens des rangées de mailles forme, seulement dans chaque deuxième ligne, des mailles flottées (F) et qu'un flotté (K) du fil de trame (5) ainsi que l'emplacement d'attache qui suit sont décalés sur le côté autour d'une maille de manière à créer un flotté (K) du fil de trame (5) sur deux ou trois mailles.

2. Tricot circulaire selon la revendication 1, **caractérisé en ce que** les compartiments (2) sont réalisés de manière à présenter une forme de losange ou une forme de nid d'abeilles.

3. Utilisation d'un tricot circulaire selon l'une quelconque des revendications précédentes servant à fabriquer un article de compression aux fins de la thérapie par compression de lipoedèmes ou d'oedèmes lymphatiques, d'ulcères veineux de la jambe ou aux fins de la cicatrisation.

4. Article de compression, en particulier bas de compression ou manchette de compression servant à la thérapie par compression, fabriqué à partir d'un tricot circulaire selon l'une quelconque des revendications 1 ou 2.
